(19) Europäisches Patentamt
European Patent Office
Office européen des brevets

(11) EP 1 486 479 A1

(12) EUROPEAN PATENT APPLICATION

(43) Date of publication:
15.12.2004 Bulletin 2004/51

(21) Application number: 04253380.2

(22) Date of filing: 07.06.2004

(51) Int Cl.[7]: C07B 39/00, C07B 37/04, C07B 41/00, C07B 43/00, C07D 307/24, C07D 307/14, C07C 17/20, C07C 253/14, C07C 255/19, C07C 331/18, C07D 307/32, C07C 319/14, C07C 45/63, C07C 205/02, C07C 209/08, C07F 9/06

(84) Designated Contracting States:
AT BE BG CH CY CZ DE DK EE ES FI FR GB GR HU IE IT LI LU MC NL PL PT RO SE SI SK TR
Designated Extension States:
AL HR LT LV MK

(30) Priority: 12.06.2003 JP 2003168116

(71) Applicant: MITSUI CHEMICALS, INC.
Tokyo (JP)

(72) Inventors:
- Funaki, Setsuko, c/o Mitsui Chemicals, Inc. Sodegaura-City, Chiba 299-0265 (JP)
- Taniguchi, Yoshiteru, c/o Mitsui Chemicals, Inc. Sodegaura-City, Chiba 299-0265 (JP)
- Nobori, Tadahito, c/o Mitsui Chemicals, Inc. Sodegaura-City, Chiba 299-0265 (JP)
- Yamamoto, Yoshihiro, c/o Mitsui Chemicals, Inc. Sodegaura-City, Chiba 299-0265 (JP)
- Hara, Isao, c/o Mitsui Chemicals, Inc. Sodegaura-City, Chiba 299-0265 (JP)
- Hayashi, Takaomi, c/o Mitsui Chemicals, Inc. Sodegaura-City, Chiba 299-0265 (JP)
- Mizutani, Kazumi, c/o Mitsui Chemicals, Inc. Sodegaura-City, Chiba 299-0265 (JP)
- Kiyono, Shinji, c/o Mitsui Chemicals, Inc. Sodegaura-City, Chiba 299-0265 (JP)

(74) Representative: Paget, Hugh Charles Edward et al
Mewburn Ellis LLP
York House
23 Kingsway
London WC2B 6HP (GB)

# (54) Method for producing organic compounds by substituting halogen atoms

(57) The invention pertains to a method in which a halogen atom of an organic compound is replaced with a group derived from a nucleophilic agent, at high yield and high efficiency, by the following method which includes a step of reacting the nucleophilic agent with an organic material having a halogen atom bonded to a carbon atom having four σ bonds, more specifically: a method for producing an organic compound having Q, the method including a step of reacting a compound represented by general formula (2) with an organic starting material having at least one halogen atom bonded to a carbon atom having four σ bonds so as to replace the halogen atom in the organic starting material with Q:

$$MQ_a \qquad (2)$$

(wherein M represents an alkali metal atom, an alkali earth metal atom, or a rare earth metal atom; Q represents a moiety of an inorganic acid or an active hydrogen compound derived by eliminating a proton, wherein Q is a halogen atom different from the halogen atom in the organic starting material having the halogen atom bonded to the carbon atom having the four σ bonds; and a represents an integer of 1 to 3) in the presence of a compound represented by general formula (1)

EP 1 486 479 A1

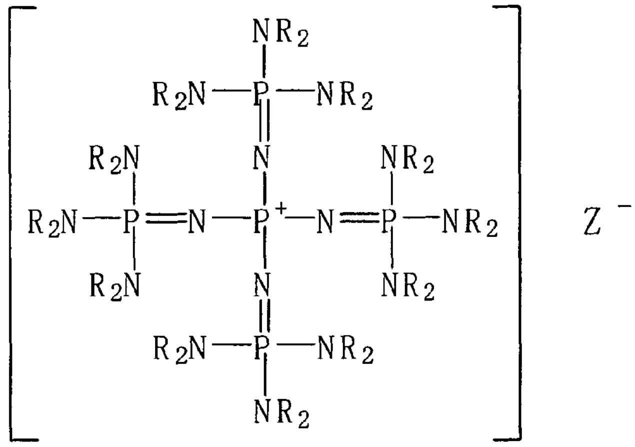

(1)

(wherein $Z^-$ represents an anion derived by eliminating a proton from an inorganic acid or an active hydrogen compound; $R_2$ is the same or different; $R_2$ each independently represent a $C_1$-$C_{10}$ hydrocarbon group or two $R_2$ on the same nitrogen atom may be bonded with each other to form a ring with the nitrogen atom).

## Description

BACKGROUND OF THE INVENTION

1. Technical Field of the Invention

The present invention relates to a method for producing an organic compound suitable for use in products, such as industrial chemicals, polymeric materials, pharmaceutical products, and agricultural chemicals, and intermediates therefor. In this method, an organic material having a halogen atom bonded to a carbon atom having four σ bonds is used as the starting material, and the halogen atom of the organic material is replaced with various nucleophilic agents.

2.Description of the Related Art

Among effective methods for producing various organic compounds, a method of utilizing nucleophilic substitution, in which halogen atoms bonded to carbon atoms are replaced with groups derived from nucleophilic agents, is known in the art.

Nucleophilic substitution has drawbacks in that the reaction takes a long time depending on the halogenated compound used, that an expensive non-protonic polar solvent is necessary to dissolve nucleophilic agents having low solubility to organic solvents, and that large amounts of nucleophilic agents are necessary.

Various catalysts have been developed to overcome these drawbacks. For example, methods that use phosphazenium compound as the catalyst (e.g., Japanese Unexamined Patent Application Publication No. 2002-003427), methods that use crown ether as the catalyst (e.g., Journal of Organic Chemistry 43 (1978) 1017-1018), methods that use ion exchange resin as the catalyst (e.g., Journal of Organic Chemistry 50 (1985) 4388-4390), methods that use quaternary ammonium salts as the catalyst (e.g., Journal of Organic Chemistry 50 (1985) 879-882), and methods that use quaternary phosphonium salts, which contain hydrocarbon groups and the like, as the catalyst (e.g., Tetrahedron Letters 27 (1986) 6067-6070) are known in the art.

However, when crown ethers and ion exchange resin are used, it takes a long time, e.g., one to two days, to carry out the reaction depending on the halogenated compound used as the reaction substrate. Thus, the efficiency is low. When quaternary ammonium salts and quaternary phosphonium salts containing hydrocarbons and the like are used, the salts may become decomposed depending on the type of nucleophilic agent or the reaction temperature employed. Thus, recycling is difficult, and the allowable reaction conditions are limited, which is problem.

SUMMARY OF THE INVENTION

An object of the present invention is to provide a method for manufacturing an organic compound, in which a halogen atom is replaced with a group derived from a nucleophilic agent, at high yield and high efficiency, the method including a step of reacting the nucleophilic agent with an organic starting material having a halogen atom bonded to a carbon atom having four σ bonds.

The Inventors have conducted extensive investigations to solve the problem described above and found that the reaction between a nucleophilic agent and an organic material having halogen atoms bonded to carbon atoms having four σ bonds can be smoothly carried out in the presence of a compound (Chemical 1) represented by general formula (1) below and that a target organic compound produced by substituting the halogen atoms with groups derived from the nucleophilic agent can be produced at high yield and high efficiency. Thus, the inventors have made the present invention.

In particular, the present invention provides:

(1) A method for producing an organic compound having Q, the method including reacting a compound represented by general formula (2) with an organic starting material having at least one halogen atom bonded to a carbon atom having four σ bonds so as to replace the halogen atom in the organic starting material with Q:

$$MQ_a \qquad (2)$$

(wherein M represents an alkali metal atom, an alkali earth metal atom, or a rare earth metal atom; Q represents a moiety of an inorganic acid or an active hydrogen compound derived by eliminating a proton, wherein Q is a halogen atom different from the halogen atom in the organic starting material having the halogen atom bonded to

the carbon atom having the four σ bonds; and a represents an integer of 1 to 3) in the presence of a compound represented by general formula (1)

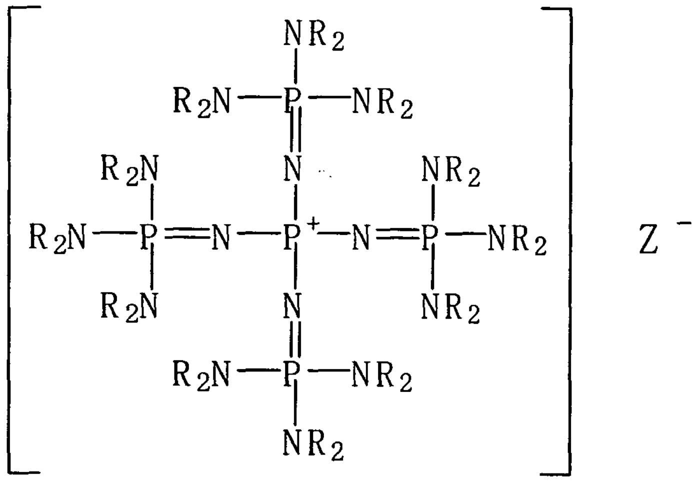

(1)

(wherein $Z^-$ represents an anion derived by eliminating a proton from an inorganic acid or an active hydrogen compound; $R_2$ is the same or different; $R_2$ each independently represent a $C_1$-$C_{10}$ hydrocarbon group or two $R_2$ on the same nitrogen atom may be bonded with each other to form a ring with the nitrogen atom).

(2) The method described in (1) above, in which the organic starting material having the halogen atom bonded with the carbon atom having the four σ bonds is represented by general formula (3), and the organic compound having Q is represented by general formula (4):

$$B\text{-}X \qquad (3)$$

(wherein X represents a halogen atom, and B represents an organic group),

$$B\text{-}Q \qquad (4)$$

(wherein B and Q are the same as above).

(3) The method described in (2) above, in which B in the compound represented by general formula (3) is selected from a $C_1$-$C_{12}$ straight or branched alkyl group, a group represented by general formula (5), a group represented by general formula (6), a group represented by general formula (7), a group represented by general formula (8), a group represented by general formula (9), or a $C_1$-$C_{12}$ straight or branched alkyl group substituted with a group selected from Substituent Group α:

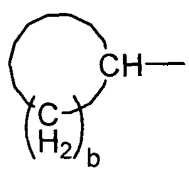

(5)

(wherein b is an integer of 2 to 9),

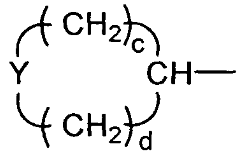

(6)

(wherein Y represents an oxygen atom, a sulfur atom, or NR'; c and d represent integers satisfying the relationship c + d = 3 to 6; and R' in NR' represents a hydrogen atom or a methyl group),

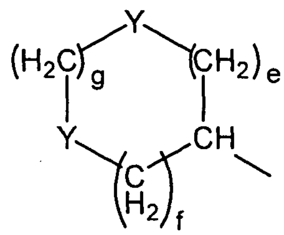

(7)

(wherein Y represents an oxygen atom, a sulfur atom, or NR'; e, f, and g are integers satisfying the relationship e + f + g = 2 to 5; and R' in NR' represents a hydrogen atom or a methyl group),

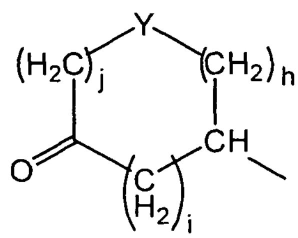

(8)

(wherein Y represents an oxygen atom, a sulfur atom, or NR'; h, i and j represent integers satisfying the relationship h + i + j = 2 to 5; and R' in NR' represents a hydrogen atom or a methyl group),

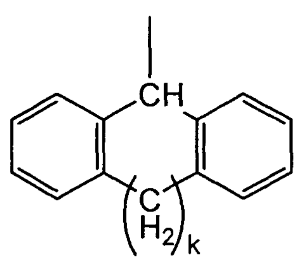

(9)

(wherein k represents an integer between 0 and 2),
[Substituent Group $\alpha$]
groups represented by general formula (5) to (9); $C_2$-$C_{10}$ alkenyl groups; $C_2$-$C_{10}$ alkynyl groups; $C_6$-$C_{12}$ aryl groups; acyl groups having a total of 2 to 10 carbon atoms including carbon atoms of carbonyl groups; acyloxy groups having a total of 2 to 10 carbon atoms including carbon atoms of carbonyl groups; alkoxycarbonyl groups having a total of 2 to 10 carbon atoms including carbon atoms of carbonyl groups; arylcarbonyl groups having a total of 7 to 10 carbon atoms including carbon atoms of carbonyl groups; alkoxycarbonylalkyl groups having a total of 3 to 10 carbon atoms including carbon atoms of carbonyl groups; nitro groups; and cyano group.

(4) The method according to (3) above, in which all $R_2$ in the compound represented by general formula (1) in (1) above are $C_1$-$C_2$ alkyl groups or every pair of $R_2$ bonded on the same nitrogen atom forms a ring as a $C_4$-$C_5$ alkylene group.

(5) The method described in (3) above, in which the compound from which $Z^-$ in general formula (1) is derived is a hydrogen halide.

(6) The method described in (3) above, wherein Q in the compound represented by general formula (2) in (1) above is a moiety derived by eliminating proton of one selected from hydrogen halides, hydrogen cyanides, hydrogen azides, thiocyanic acids, malonic esters, acetoacetic esters, cyanoacetic esters, water, carboxylic acids having a total of 1 to 20 carbon atoms including carbon atoms of carbonyl groups, $C_1$-$C_{20}$ alcohols, $C_6$-$C_{20}$ aromatic compounds having 1 to 3 hydroxyl groups, aliphatic secondary amines having a total of 2 to 20 carbon atoms, aromatic secondary amines having a total of 6 to 20 carbon atoms, $C_1$-$C_{10}$ monovalent thiols, and $C_1$-$C_{10}$ aromatic mercapto compounds.

(7) The method described in (3) above, in the compound represented by general formula (1) in (1) above, all $R_2$ in general formula (1) are methyl or ethyl groups, or every pair of $R_2$ bonded to the same nitrogen atom is a tetramethylene group; $Z^-$ is an chlorine anion or a bromine anion; in the compound represented by general formula (2) in (1) above, Q is a moiety of an active hydrogen compound derived by eliminating a proton, the active hydrogen compound being selected from Compound Group β; in the compound represented by general formula (3) in (2) above, B represents a $C_1$ to $C_{10}$ straight alkyl group; b in the group represented by general formula (5) in (3) above is 4 to 7; Y in the group represented by general formula (6) in (3) above is an oxygen atom, and c + d is 3 or 4; in the group represented by general formula (8) in (3) above, Y is an oxygen atom, h = 2, i = 0, and j = 0; k in the group represented by general formula (9) in (3) above, k is zero; or $C_1$-$C_{10}$ straight alkyl groups substituted with groups selected from Substituent Group α in (3) above consists of Substituent Group γ:

[Compounds Group β]

hydrogen fluoride, hydrogen iodide, hydrogen cyanide, hydrogen azide, thiocyanic acid, diethyl malonate, water, acetic acid, methanol, phenol, diethylamine, nitrous acid, and n-butylthiol;

[Substituent Group γ]

tetrahydrofuryl group, 2-oxotetrahydrofuryl group, ethynyl group, phenyl group, acetyl group, pivalyl group, benzoyl group, butyryloxy group, methoxycarbonyl group, ethoxycarbonyl group, methoxycarbonylmethyl group, nitro group, and cyano group.

(8) The method according to one of (1) to (7), in which the reaction is conducted in the presence of a compound represented by general formula (10):

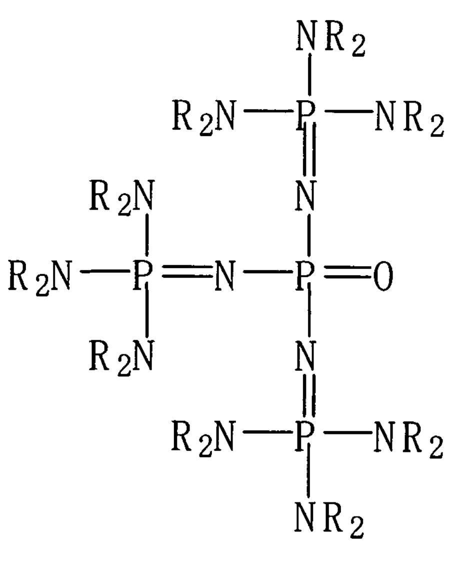

(10)

(wherein $R_2$ is the same or different; $R_2$ each independently represent a $C_1$-$C_{10}$ hydrocarbon group or two $R_2$ on the same nitrogen atom may be bonded with each other to form a ring with the nitrogen atom).

(9) The method according to one of (3) to (8), in which, in the compound represented by general formula (10) in (8) above, all $R_2$ are $C_1$-$C_2$ alkyl groups, or every pair $R_2$ bonded to the same nitrogen atom forms a ring as a $C_4$-$C_5$ alkylene group.

The present invention can provide a method for producing organic compounds by substituting halogen atoms of a halogenated compound starting material with various substituents in the presence of a compound represented by general formula (1). This method is industrially advantageous over conventional methods.

DESCRIPTION OF THE PREFERRED EMBODYMENTS

General formula (1) representing the compound of the present invention is a canonical structure formula in which a positive charge of a phosphazenium cation localizes on the phosphorus atom at the center (i.e., general formula (1)). However, the compound can also be represented by other canonical structures. In general, the actual positive charge is nonlocalized.

Examples of the compounds from which $Z^-$ is derived include inorganic acids. Examples of the inorganic acid include hydrogen halides such as hydrogen fluoride, hydrogen chloride, hydrogen bromide, hydrogen iodide, and bromoform; sulfuric acid; perchloric acid; hexafluorophosphoric acid; tetrafluoroboric acid; hydrogen cyanide; thiocyanic acid; and hydrogen azide.

Examples of the compounds from which $Z^-$ is derived also include active hydrogen compounds. Active hydrogen compounds are not particularly limited as long as they can give anions by elimination of protons. Examples of the active hydrogen compounds include compounds having carbon atoms bonded with active hydrogen atoms, compounds having oxygen atoms bonded with active hydrogen atoms, compounds having nitrogen atoms bonded with active hydrogen atoms, and compounds having sulfur atoms bonded with active hydrogen atoms.

Examples of the compounds having carbon atoms bonded with active hydrogen atoms include compounds in which a carbon atom having at least one hydrogen atom is bonded with a cyano group, a nitro group, a phenyl group, an acyl group, an alkoxycarbonyl group, an alkenyl group, or an alkynyl group, and compounds in which an active hydrogen atom is directly bonded with an alkenyl or alkynyl group.

Specific examples of the compounds in which carbon atoms are bonded with active hydrogen atoms include cyano-group-containing compounds such as acetonitrile, n-valeronitrile, n-butyronitrile, adiponitrile, malononitrile, phenylacetonitrile, succinonitrile, 3-methoxypropionitrile, 4-methylbenzyl cyanide, 2-nitrophenylacetonitrile, 4-methoxyphenylacetonitrile, glutaronitrile, 3-phenylpropionitrile, cyclopentenylacetonitrile, isopropylidenemalononitrile, benzoylacetonitrile, and pivaloylacetonitrile; alkoxycarbonyl-group-containing compounds such as methyl acetate, n-propyl acetate, isopropyl acetate, 2-methoxyethyl acetate, tert-butyl acetate, phenyl acetate, ethyl n-butyrate, ethyl propionate, benzyl acetate, n-amyl acetate, acetic anhydride, ethyl isovalerate, methyl levulinate, and 2-methylcyclohexyl acetate; nitro-group-containing compounds such as nitroethane, 1-nitropropane, 1-nitrobutane, methyl 4-nitrobutyrate, methyl nitroacetate, nitrocyclopentane, dinitromethane, and 1,1-dinitroethane; acyl-group-containing compounds such as 2-butanone, 2-hexanone, 2-heptanone, 4,4-dimethyl-2-pentanone, methoxyacetone, cyclohexylmethylketone, acetophenone, benzylacetone, acetylacetone, 1-benzoylacetone, 1,3-cyclopentanedione, 1,3-cyclohexanedione, dibenzoylmethane, anthrone, 1,3-indanedione, and 3,5-heptanedione; alkenyl-group-containing compounds such as 2,4-dimethyl-2-pentene, 2-methyl-1-phenylpropene, 1-methyl-1-cyclopentene, 6,6-dimethylfulvene, 1,2,3,4,5-pentamethylcyclopentadiene, 1,3,5,5-tetramethyl-1,3-cyclohexadien; alkynyl-group-containing compounds such as 1-butyne, 2-butyne, 1-phenyl-1-propyne, 2-pentyne, methylpropargyl ether, 4-methyl-2-pentyne, 2,4-hexadiyne, 2-butynyl acetate, and acetylene; phenyl group-containing compounds such as diphenylmethane, triphenylmethane, xanthene, 9,10-dihydroanthracene, fluorene, 2,7-dinitrofluorene, 4,4'-difluorodiphenylmethane, 4-benzylbiphenyl, 4-nitrodiphenylmethane, and 4,4'-dinitrodiphenylmethane; malonic esters such as dimethyl malonate, diethyl malonate, di-n-butyl malonate, di-tert-butyl malonate, and benzylmethyl malonate; acetoacetic esters such as methyl acetoacetate, ethyl acetoacetate, n-butyl acetoacetate, and sec-butyl acetoacetate; and cyanoacetic esters such as methyl cyanoacetate, ethyl cyanoacetate, n-butyl cyanoacetate, isobutyl cyanoacetate, tert-butyl cyanoacetate, 2-ethylhexyl cyanoacetate, and benzyl cyanoacetate.

Examples of the compounds having oxygen atoms bonded with active hydrogen atoms include water, carboxylic acids having a total of 1 to 20 carbon atoms including carbon atoms of carbonyl groups, carbamic acids having a total of 2 to 20 carbon atoms including carbon atoms of carbonyl groups; $C_1$-$C_{20}$ sulfonic acids; $C_1$-$C_{20}$ alcohols; saccharides and derivatives thereof; $C_6$-$C_{20}$ aromatic compounds having hydroxy groups; and polyalkylene oxides.

Examples of the carboxylic acids having a total of 1 to 20 carbon atoms including carbon atoms of carbonyl groups include monocarboxylic acids such as formic acid, acetic acid, butyric acid, isobutyric acid, lauric acid, stearic acid, oleic acid, phenylacetic acid, dihydrocinnamic acid, cyclohexanecarboxylic acid, benzoic acid, and 2-carboxynaphthalene; and polyvalent carboxylic acids such as oxalic acid, malonic acid, succinic acid, maleic acid, fumaric acid, adipic acid, butanetetracarboxylic acid, isophthalic acid, terephthalic acid, trimellitic acid, and pyromellitic acid.

Examples of the carbamic acids having a total of 2 to 20 carbon atoms including carbon atoms of carbonyl groups include N,N-diethylcarbamic acid, N-carboxy pyrrolidone, N-carboxy aniline, and N,N'-dicarboxy-2,4-toluenediamine.

Examples of the $C_1$-$C_{20}$ sulfonic acids include methanesulfonic acid, ethanesulfonic acid, p-toluenesulfonic acid, 4-ethylbenzenesulfonic acid, picrylsulfonic acid, 4-nitrobenzenesulfonic acid, 3-(N-morpholino)propanesulfonic acid, 2-morpholinoethanesulfonic acid, 2-naphthalenesulfonic acid, 4,4'-biphenyldisulfonic acid, 4-nitrotoluene-2-sulfonic acid, and 3-pyridinesulfonic acid.

Examples of the $C_1$-$C_{20}$ alcohols include monovalent alcohols such as methanol, ethanol, n-butyl alcohol, sec-butyl alcohol, tert-butyl alcohol, n-octyl alcohol, lauryl alcohol, cyclopentanol, cyclohexanol, allyl alcohol, benzyl alcohol, 1-phenylethyl alcohol, triphenylcarbinol, and cinnamyl alcohol; and polyalcohols such as ethylene glycol, propylene glycol, diethylene glycol, dipropylene glycol, 1,3-propanediol, 1,4-butanediol, 1,6-hexanediol, 1,4-cyclohexanediol, trimethylolpropane, glycerin, diglycerin, pentaerythritol, and dipentaerythritol.

Examples of the saccharides and derivatives thereof include glucose, sorbitol, dextrose, fructose, and sucrose.

Examples of the $C_6$-$C_{20}$ aromatic compounds having hydroxy groups include phenol, catechol, resorcinol, hydroquinone, 1-naphthol, 2-naphthol, 1,2-dihydroxynaphthalene, 2,6-dihydroxynaphthalene, o-cresol, m-cresol, p-cresol, 2,4,6-trimethylphenol, 4-nitrophenol, 4-methoxyphenol, anthrarobin, 9-phenanthrol, 1-hydroxypyrene, and bisphenol A.

Examples of the polyalkylene oxides include polyethylene oxide, polypropylene oxide, and copolymers thereof.

Examples of the compounds having nitrogen atoms bonded with active hydrogen atoms include $C_1$-$C_{20}$ aliphatic primary amines, $C_6$-$C_{20}$ aromatic primary amines, aliphatic secondary amines having a total of 2 to 20 carbon atoms, aromatic secondary amines having a total of 6 to 20 carbon atoms, polyvalent amines having primary or secondary amine and having a total of 2 to 20 carbon atoms, saturated or unsaturated cyclic secondary amines having a total of 4 to 20 carbon atoms, cyclic polyvalent amines including secondary amines and having a total of 4 to 20 carbon atoms, acid amides having a total of 2 to 20 carbon atoms including carbon atoms of carbonyl groups obtained from primary or secondary amines, five- to seven-membered cyclic amides, and imides of dicarboxylic acid having a total of 4 to 10 carbon atoms including carbon atoms of carbonyl groups.

Examples of $C_1$-$C_{20}$ aliphatic primary amines include methylamine, ethylamine, n-butylamine, isobutylamine, sec-butylamine, tert-butylamine, and cyclohexylamine.

Examples of the $C_6$-$C_{20}$ aromatic primary amines include benzylamine, β-phenylethylamine, aniline, o-toluidine, m-toluidine, and p-toluidine.

Examples of the aliphatic secondary amines having a total of 2 to 20 carbon atoms include dimethylamine, methyl ethyl amine, diethylamine, ethyl-n-butylamine, methyl-sec-butylamine, dipentylamine, and dicyclohexylamine.

Examples of the aromatic secondary amines having a total of 6 to 20 carbon atoms include N-methylaniline and diphenylamine.

Examples of the polyvalent amines having primary or secondary amino groups and having a total of 2 to 20 carbon atoms include ethylene diamine, di(2-aminoethyl)amine, hexamethylenediamine, 4,4'-diaminodiphenylmethane, tri(2-aminoethyl)amine, N,N'-dimethylethylenediamine, N,N'-diethylethylenediamine, and di(2-methylaminoethyl) amine.

Examples of the saturated or unsaturated cyclic secondary amines having a total of 4 to 20 carbon atoms include pyrrolidine, piperidine, morpholine, 1,2,3,4-tetrahydroquinoline, 3-pyrroline, pyrrole, indole, carbazole, imidazole, pyrazole, and purine.

Examples of the cyclic polyvalent amines having a total of 4 to 20 carbon atoms and secondary amino groups include piperazine, pyrazine, and 1,4,7-triazacyclononane.

Examples of the amides having a total of 2 to 20 carbon atoms including carbon atoms of carbonyl groups obtained from primary or secondary amines include acetamide, propionamide, N-methylpropionamide, N-methylbenzamide, and N-ethylstearamide.

Examples of the five- to seven-membered cyclic amides include 2-pyrrolidone and ε-caprolactam.

Examples of the imides of dicarboxylic acids having 4 to 10 carbon atoms including carbon atoms of carbonyl groups include succinimide, maleinimide, and phthalimide.

Examples of the compounds having sulfur atoms bonded to active hydrogen atoms include monothiols having 1 to 10 carbon atoms, polyvalent thiols having 1 to 10 carbon atoms, and aromatic mercapto compounds having 1 to 10 carbon atoms.

Examples of the monothiols having 1 to 10 carbon atoms include methanethiol, ethanethiol, n-butanethiol, tert-butanethiol, hexanethiol, decanethiol, cyclopentyl mercaptan, and cyclohexyl mercaptan.

Examples of the polyvalent thiols having 1 to 10 carbon atoms include 1,2-ethanedithiol, 1,3-propanedithiol, 2,3-butanedithiol, 1,6-hexanedithiol, 1,2,3-propanetrithiol, and 2,3-di(mercaptomethyl)-1,4-butanedithiol.

Examples of the aromatic mercapto compounds having 1 to 10 carbon atoms include thiophenol, o-thiocresol, thionaphthol, and 1,2-benzenedithiol.

Among the compounds from which $Z^-$ is derived, hydrogen halides such as hydrogen fluoride, hydrogen chloride, hydrogen bromide, hydrogen iodide, and bromoform, tetrafluoroborate, and hexafluorophosphate are preferred. Hydrogen chloride, hydrogen bromide, and hydrogen iodide are more preferred.

$R_2$ in general formula (1) is the same or different. $R_2$ each independently represent a $C_1$-$C_{10}$ hydrocarbon group or two $R_2$ on the same nitrogen atom may be bonded with each other to form a ring with the nitrogen atom.

Examples of the $C_1$-$C_{10}$ hydrocarbon group include aliphatic and aromatic hydrocarbon groups such as methyl, ethyl, n-butyl, sec-butyl, tert-butyl, 2-butenyl, 1-pentyl, 2-methyl-1-butyl, tert-pentyl, 3-methy-2-butyl, neopentyl, n-hexyl, 4-methyl-2-pentyl, cyclopentyl, cyclohexyl, 1-octyl, 2-octyl, 2-ethyl-1-hexyl, 1,1-dimethyl-3,3-dimethylbutyl (a.k.a. tert-octyl), phenyl, 4-toluyl, benzyl, 1-phenylethyl and 2-phenylethyl. Among these, methyl, ethyl, n-propyl, isopropyl, tert-butyl, tert-pentyl and 1,1-dimethyl-3,3-dimethylbutyl are preferred. Methyl is more preferred.

In general formula (1), when two $R_2$ on the same nitrogen atom bonded with each other to form a ring structure, the ring preferably contains a divalent straight hydrocarbon group having a $C_4$-$C_6$ main chain and the nitrogen atom bonded to that hydrocarbon group (the ring formed becomes a five- to seven-membered ring containing the nitrogen atom). The hydrocarbon group is preferably selected from a tetramethylene group, a pentamethylene group, a hexamethylene group, and groups having these main chains substituted with alkyl groups such as methyl and ethyl. Tetramethylene and pentamethylene are more preferable.

Among the groups having two $R_2$ bonded on the same nitrogen atom in general formula (1), either all or part of such groups may have the two $R_2$ bonded with each other to form a ring. When part of the groups have the two $R_2$ bonded with each other to form a ring, the $R_2$ of the remaining groups that do not form a ring are preferably those described above as the examples of $R_2$ when $R_2$ independently represent hydrocarbon groups.

One or a combination of a plurality of types of compounds represented by general formula (1) can be used.

Compounds represented by general formula (1) can be produced by a method disclosed in Japanese Unexamined Patent Application Publication No. 10-77289 or a method comparable to this method.

Compounds represented by general formula (1) used in the method of the present invention can be prepared in advance by the above-described methods. Alternatively, the compounds may be prepared from suitable starting materials in the reaction system.

In general formula (2), M represents an alkali metal atom, an alkali earth metal atom, or a rare earth metal atom.

For example, the alkali metal atom is lithium, sodium, potassium, cesium, or rubidium.

For example, the alkali earth metal atom is magnesium, calcium, strontium, or barium.

For example, the rare earth metal atom is cerium, praseodymium, neodymium, or samarium.

Among these, lithium, sodium, potassium, cesium, and rubidium are preferred. Sodium and potassium are more preferred.

In general formula (2), Q represents a moiety of an inorganic acid or an active hydrogen compound derived by eliminating a proton. Q is never the same halogen atom as the halogen atom in an organic starting material described below replaced with Q.

In general formula (2), the inorganic acid or the active hydrogen compound from which Q is derived may include a plurality of active hydrogen atoms. The active hydrogen atoms may be bonded to the same or different atoms in the inorganic acid or the active hydrogen compound.

Examples of the inorganic acid and the active hydrogen compounds from which Q is derived are the same as those described above as the compounds from which $Z^-$ in general formula (1) is derived; accordingly detailed description is not provided here. Preferable examples of the active hydrogen compound from which Q is derived include hydrogen halides such as hydrogen fluoride, hydrogen chloride, hydrogen bromide, or hydrogen iodide; hydrogen cyanide; hydrogen azide; thiocyanic acid; water; sulfuric acid; nitrous acid; compounds having cyano group such as phenylacetonitrile, malononitrile, benzoylacetonitrile, and pivaloylacetonitrile; compounds having nitro group such as dinitromethane, methyl nitroacetate, and ethyl nitroacetate; groups having acyl groups such as acetylacetone, 1-benzoylacetone, 1,3-cyclopentadione, and 1,3-cyclohexanedione; groups having alkoxycarbonyl groups such as methyl acetate, ethyl acetate, and phenyl acetate; compounds having alkenyl groups such as cyclopentadiene, 2-methyl-1-phenylpropene; compounds having phenyl groups such as diphenylmethane, triphenylmethane, and fluorene; dimethyl malonate, diethyl malonate, methyl acetoacetate, ethyl acetoacetate, methyl cyanoacetate, ethyl cyanoacetate, carboxylic acids having a total of 1 to 20 carbon atoms including carbon atoms of carbonyl groups, such as formic acid, acetic acid, lauric acid, stearic acid, phenylacetic acid, dihydrocinnamic acid, cyclohexanecarboxylic acid, benzoic acid, and 2-carboxynaphthalene; $C_1$-$C_{20}$ sulfonic acids such as methanesulfonic acid, benzenesulfonic acid, p-toluenesulfonic acid, trifluoromethanesulfonic acid, 4-nitrobenzenesulfonic acid, 3-(N-morpholino)propanesulfonic acid, 2-naphthalenesulfonic acid, and 3-pyridinesulfonic acid; $C_1$-$C_{20}$ alcohols such as methanol, n-butyl alcohol, sec-butyl alcohol, tert-butyl alcohol, cyclopentanol, allyl alcohol, crotyl alcohol, benzyl alcohol, triphenylcarbinol, and cinnamyl alcohol; $C_6$-$C_{20}$ aromatic compounds having hydroxyl groups, such as phenol, hydroquinone, 2-naphthol, 2,6-dihydroxynaphthalene, 4-nitrophenol, and bisphenol A; aliphatic secondary amines having a total of 2 to 20 carbon atoms such as dimethylamine, methyl ethyl amine, ethyl-n-butylamine, methyl-sec-butylamine, dipentylamine, and dicyclohexylamine; aromatic secondary amines having a total of 6 to 20 carbon atoms, such as N-methylaniline and diphenylamine; $C_1$-$C_{10}$ monovalent thiols such as methanethiol, n-butanethiol, tert-butanethiol, decanethiol, cyclopentyl mercaptan, and cyclohexyl mercaptan; and aromatic mercapto compounds such as thiophenol, o-thiocresol, thionaphthol, and 1,2-benzenedithiol.

Among these, preferred compounds are hydrogen fluoride, hydrogen iodide, hydrogen cyanide, hydrogen azide, thiocyanic acid, water, nitrous acid, malononitrile, benzoylacetonitrile, pivaloylacetonitrile, acetylacetone, 1-benzoylacetone, dinitromethane, dimethyl malonate, diethyl malonate, methyl acetoacetate, ethyl acetoacetate, methyl cyanoacetate, ethyl cyanoacetate, cyclopentadiene, diphenylmethane, triphenylmethane, fluorene, and $C_1$-$C_{20}$ carboxylic acids such as formic acid, acetic acid, propionic acid, butyric acid, isobutyric acid, lauric acid, stearic acid, oleic acid, phenylacetic acid, dihydrocinnamic acid, cyclohexanecarboxylic acid, benzoic acid, 4-methylbenzoic acid, and 2-carboxynaphthalene; $C_1$-$C_{20}$ alcohols such as methanol and ethanol; $C_6$-$C_{20}$ aromatic compounds having hydroxy groups, such as phenol, 2-naphthol, 2,6-dihydroxynaphthalene, and bisphenol A; aliphatic secondary amines having a total of 2 to 20 carbon atoms, such as dimethylamine, methyl ethyl amine, ethyl-n-butylamine, methyl-sec-butylamine, dipentylamine, and dicyclohexylamine; aromatic secondary amines having 6 to 20 carbon atoms, such as N-methylaniline and diphenylamine; monovalent thiols such as methanethiol, ethanethiol, n-butanethiol, tert-butanethiol, hexanethiol, decanethiol, cyclopentyl mercaptan, and cyclohexyl mercaptan; and aromatic mercapto compounds such as thiophenol, o-thiocresol, thionaphthol, and 1,2-benzenedithiol. Compounds selected from Compound Group β are particularly preferred.

In general formula (2), a is an integer of 1 to 3. Preferably, a is 1.

Compounds represented by general formula (2) used in the method of the present invention may be prepared in advance or may be prepared from suitable starting materials in the reaction system.

In the present invention, the organic starting material having a halogen atom bonded to a carbon atom having four σ bonds is defined as a compound having a halogen atom bonded with a carbon atom bonded with four atoms through σ bonds and located at a particular position in a molecule. When this compound is reacted with the compound represented by general formula (2) in the presence of the compound represented by general formula (1), an organic compound derived by substituting all or some of the halogen atoms in the organic starting material with Q is produced. Hereinafter, the organic staring material having the halogen atom bonded with the carbon atom having four σ bonds is simply referred to as the " halogenated compound ".

The carbon atom bonded with the halogen atom in the halogenated compound may be bonded with substituents other than the halogen as long as an organic compound can be prepared by substituting all or part of the halogen atoms in the halogenated compound with Q as a result of the reaction between the halogenated compound and the compound represented by general formula (2) in the presence of the compound represented by general formula (1).

When a plurality of halogen atoms exists in the halogenated compound, an organic compound in which all or part of the halogen atoms are replaced with Q as a result of the reaction between the halogenated compound and the compound represented by general formula (2) in the presence of the compound represented by general formula (1) can be obtained.

Examples of the halogenated compound that satisfies the above-described definition include compounds in which a predetermined number of hydrogen atoms bonded with carbon atoms are replaced with halogen atoms, the carbon atoms being contained in aliphatic saturated hydrocarbons, alicyclic hydrocarbons, hydrogenated condenced polycyclic hydrocarbons, bridged ring aliphatic hydrocarbons, aliphatic heterocyclic compounds having an oxygen atom, a nitrogen atom, or a sulfur atom.

Examples of the halogenated compound are organic compounds having 2 to 6 halogen atoms. Particular examples of the halogenated compound include compounds having two halogen atoms, such as 1,2-dichloroethane, 1-bromo-2-chloropropane, 1,2-dichloro-2-methylpropane, 1,2-dichloro-3-butene, methyl 2,3-dichloropropionate, 2,3-dichloropropionitrile, 1,2-dichlorocyclohexane, 2,3-dichloronorbornane, 2,3-dichloro-1,4-dioxane, (1,2-dichloroethyl)benzene, and 1,2-dichloroindane; compounds having four halogen atoms, such as 1,2,3,4-tetrachlorobutane, 1,2,3,4-tetrachloro-1,2,3,4-tetrahydronaphthalene; and compounds having six halogen atoms such as 1,2,3,4,5-pentabromo-6-chlorocyclohexane. However, the halogenated compound is preferably an organic compound having one halogen atom.

The organic compounds having one halogen atom can be specifically represented by general formula (3).

In general formula (3), X is fluorine, chlorine, bromine, or iodine. Among these, chlorine, bromine, and iodine are particularly preferred.

In general formula (3), B represents an organic group. The organic group is the moiety of an organic compound having one halogen atom after elimination of the halogen atom in which a halogen atom is eliminated from the organic compound that contains one halogen atom and that satisfies the definition of the halogen compound described above. Examples of B in general formula (3) include straight or branched alkyl groups that may include substituents, cycloalkyl

groups, hydrogenated condensed polycyclic hydrocarbons, bridge ring aliphatic hydrocarbons, aliphatic heterocyclic groups, and aliphatic heterocyclic groups having carbonyl groups in the rings.

Examples of the substituents that may be included in these groups include $C_2$-$C_{12}$ alkenyl groups, $C_2$-$C_{12}$ alkynyl groups, $C_6$-$C_{20}$ aryl groups, $C_1$-$C_{12}$ alkoxy groups, $C_6$-$C_{20}$ aryloxy groups, formyl groups, acyl groups having a total of 2 to 12 carbon atoms including carbon atoms of carbonyl groups, arylcarbonyl groups having a total of 7 to 20 carbon atoms including carbon atoms of carbonyl groups, amino group, $C_1$-$C_{20}$ alkylamino and dialkylamino groups, cyano group, cyanate group, isocyanate groups, thiocyanate group, isothiocyanato group, acetylamino groups having a total of 2 to 12 carbon atoms including carbon atoms of carbonyl groups, $C_6$-$C_{30}$ arylamino and diarylamino groups, nitro group, $C_1$-$C_{12}$ alkylthio groups, $C_6$-$C_{20}$ arylthio groups, acyloxy groups having a total of 2 to 12 carbon atoms including carbon atoms of carbonyl groups, arylcarbonyloxy groups having a total of 7 to 20 carbon atoms including carbon atoms of carbonyl groups, alkoxycarbonyl groups having a total of 2 to 12 carbon atoms including carbon atoms of carbonyl groups, aryloxycarbonyl groups having a total of 7 to 20 carbon atoms including carbon atoms of carbonyl groups, alkoxycarbonyloxy groups having a total of 2 to 12 carbon atoms including carbon atoms of carbonyl groups, aryloxycarbonyloxy groups having a total of 7 to 20 carbon atoms including carbon atoms of carbonyl groups, thioformyl group, alkylthiocarbonyl groups having a total of 2 to 12 carbon atoms including carbon atoms of thiocarbonyl groups, arylthiocarbonyl groups having a total of 7 to 20 carbon atoms including carbon atoms of thiocarbonyl groups, alkylthiocarboxy groups having a total of 2 to 12 carbon atoms including carbon atoms of thiocarbonyl groups, arylthiocarboxyl groups having 7 to 20 carbon atoms including carbon atoms of thiocarbonyl groups, alkyldithiocaboxyl groups having a total of 2 to 12 carbon atoms including carbon atoms of thiocarbonyl groups, aryldithiocarboxyl groups having a total of 7 to 20 carbon atoms including carbon atoms of thiocarbonyl groups, amide group, thioamide group, alkylamide and dialkylamide groups having a total of 2 to 12 carbon atoms including carbon atoms of carbonyl groups, arylamide and diarylamide groups having a total of 7 to 20 carbon atoms including carbon atoms of carbonyl groups, alkylthioamide and dialkylthioamide groups having a total of 2 to 12 carbon atoms including carbon atoms of thiocarbonyl groups, arylthioamide and diarylthioamide groups having a total of 7 to 20 carbon atoms including carbon atoms of thiocarbonyl groups, $C_1$-$C_{12}$ alkylsulfonyl groups, $C_6$-$C_{20}$ arylsulfonyl groups, $C_1$-$C_{12}$ alkyloxysulfonyl groups, $C_6$-$C_{20}$ aryloxysulfonyl groups, $C_2$-$C_{12}$ aliphatic heterocyclic groups, and $C_5$-$C_{20}$ aromatic heterocyclic groups. Examples of the straight or branched alkyl groups that may have substituents include $C_1$-$C_{20}$ straight or branched alkyl groups that may have substituents, $C_1$-$C_{12}$ straight or branched alkyl groups that may have substituents, and $C_1$-$C_{10}$ straight or branched alkyl groups that may have substituents.

Among these straight or branched alkyl groups that may have substituents, $C_1$-$C_{12}$ straight or branched alkyl groups and $C_1$-$C_{12}$ straight or branched alkyl groups having a group selected from Substituent Group $\alpha$ are preferred. $C_1$-$C_{10}$ straight alkyl groups and $C_1$-$C_{10}$ straight alkyl groups having a group selected from Substituent Group $\gamma$ are particularly preferred.

Examples of cycloalkyl groups that may have substituents include $C_3$-$C_{10}$ cycloalkyl groups that may have substituents and groups represented by general formula (5). In general formula (5), b represents an integer of 2 to 9.

Among the cycloalkyl groups that may have substituents, groups represented by general formula (5) are preferred, and groups in which b in general formula (5) is 4 to 7 are particularly preferred.

Examples of aliphatic heterocyclic groups that may have substituents include $C_4$-$C_7$ aliphatic heterocyclic groups that may have substituents, groups represented by general formula (6), and groups represented by general formula (7).

In general formula (6), Y represents an oxygen atom, sulfur atom, or NR', and c and d represent integers that satisfy the relationship c + d = 3 to 6. R' in NR' represents a hydrogen atom or a methyl group.

In general formula (7), Y represents an oxygen atom, sulfur atom, or NR', and e, f, and g represent integers that satisfy the relationship e + f + g = 2 to 5. R' in NR' represents a hydrogen atom or a methyl group.

Among the aliphatic heterocyclic groups that may have substituents, groups represented by general formula (6) and (7) are preferred. In particular, groups represented by general formula (6), in which Y is an oxygen atom and c + d is 3 or 4, are particularly preferred.

Examples of the aliphatic heterocyclic groups having carbonyl groups in the ring that may have substituents include $C_4$-$C_7$ aliphatic heterocyclic groups and groups represented by general formula (8).

In general formula (8), Y represents an oxygen atom, sulfur atom, or NR', and h, i, and j represent integers that satisfy the relationship h + i + j = 2 to 5. R' in NR' represents a hydrogen atom or a methyl group.
Among the aliphatic heterocyclic groups having carbonyl groups in the ring that may have substituents, groups represented by general formula (8) are preferred. Groups represented by general formula (8), in which Y is an oxygen atom, h is 2, i is 0, and j is 0, are particularly preferred.

Examples of the hydrogenated condensed polycyclic hydrocarbon groups that may have substituents include $C_6$-$C_{13}$ hydrogenated condensed polycyclic hydrocarbon groups that may have substituents and groups represented by general formula (9).

In general formula (9), k represents an integer of 0 to 2.

Among the hydrogenated condensed polycyclic hydrocarbon atoms that may have substituents, groups represented by general formula (9) are preferred, and groups represented by general formula (9) in which k is 0 are particularly preferred.

Specific examples of the compounds represented by general formula (3) are presented below.

Examples of the compounds in which B in general formula (3) is a straight or branched alkyl group that may have a substituent include 2-chloropropane, 2-chlorobutane, 2-chloropentane, 3-chloropentane, 2-chlorohexane, 3-chlorohexane, 2-chloroheptane, 4-chloroheptane, 2-chlorooctane, (chloromethyl)cyclopentane, (bromomethyl)cyclohexane, 2-chloromethyl-1,3-dimethylcyclohexane, 4-chlorooctane, 3-chloro-1-propene, 1-chloro-3-methyl-2-butene, 3-chloro-5-methoxy-1-pentene, 1-chloro-2-pentyne, 3-chloro-3-methyl-1-butyne, 3-chloro-1-butyne, 1-(chloroethyl)benzene, dodecylbenzyl chloride, diphenylmethyl chloride, 1-chloro-3,3-diethoxypropane, 2-chloropropionaldehyde dimethyl acetal, 2-chloroethyl methyl ether, 1-chloro-4-(chloromethoxy)benzene, dichlorodiphenoxymethane, 3-chloro-2-butanone, 3-chloro-3-methyl-2-butanone, chloromethyl acetate, chloromethyl butyrate, 3-chloroacetylacetone, methyl 2-chloropropionate, methyl 3-chlorobutyrate, ethyl 2-chlorobutyrate, 2-chloroacetophenone, 4-methylphenacyl chloride, methyl 3-chlorobutyrate, 1-chloro-1-nitropropane, 2-chloro-2-nitropropane, 2-chloropropionitrile, 9-(chloromethyl)fluorene, 2-(N,N-bis (chloromethyl) amino) anthracene, 2-(N,N-bis(chloromethyl)amino)-7-chlorofluorene, 3-chloropropyldodecyl sulfide, chloromethylcyclohexyl sulfide, 2-chloro-N-(4H-(1,2,4)triazol-3-yl)-acetamide, 2-chloro-N-methylacetamide, 3-(chloromethyl)tetrahydrofuran, 2-(bromomethyl)tetrahydropyran, 2-chloromethyl-1, 3-dimethylcyclohexane, 2-chloromethyl-1,3-dioxolane, 2-(chloromethyl)-1,3-dioxane, 5-(bromomethyl)-2-pyrrolidinone, and 1-benzyl-5-chloromethyl-2-pyrrolidinone.

Examples of compounds in which B in general formula (3) is a cycloalkyl group that may have a substituent include chlorocyclopentane, iodocyclohexane, chlorocycloheptane, chlorocyclooctane, and 1-chloro-1-ethylcyclohexane.

Examples of the compounds in which B in general formula (3) is an aliphatic heterocyclic group that may have a substituent include 2-chlorotetrahydrofuran, 3-chlorotetrahydrofuran, 4-chlorotetrahydropyran, 4-chloro-N-methylpiperidine, 4-chlorotetrahydrothiopyran, 3-chlorotetrahydrothiophene-1,1-dioxide, 5-chloro-1,3-dioxane, 3-bromotetrahydro-2-methyl-2H-pyran, 3,4,5-triacetoxy-2-chloro-6-methyltetrahydropyran, (2-(1-bromoundecyl)-(1,3)dioxolan-4-yl)-methanol, and 2,2-dimethyl-4-bromo-1,3-dioxane.

Examples of the compounds in which B in general formula (3) is an aliphatic heterocyclic group, having a carbonyl group in the ring, that may have a substituent, include α-bromo-γ-butyrolactone, 3-bromo-1-phenyl-2-pyrrolidinone, 3-chloro-2-piperidone, and α-bromo-α-methyl-γ-butyrolactone.

Examples of the compounds in which B in general formula (3) is a hydrogenated condensed polycyclic hydrocarbon group that may have a substituent include 9-chlorofluorene and 9-bromo-2-nitrofluorene.

Examples of the compounds in which B in general formula (3) is a bridged ring aliphatic hydrocarbon group that may have a substituent include 7-chloronorbornane, 2-bromo-1,7,7-trimethylnorbornane, 1-bromoadamantane, 2-chlorobicyclo[2.2.2]octane.

The compound represented by general formula (3) is reacted with the compound represented by general formula (2) in the presence of the compound represented by general formula (1) to obtain a compound represented by general formula (4) in which the halogen atom X in general formula (3) is replaced with Q in general formula (2).

During the reaction, a solvent may be used if necessary. The solvent may be any suitable one as long as the solvent does not hinder the reaction between the compound represented by general formula (2) and the halogenated compound. Examples of the solvent include non-protonic polar solvents such as N,N-dimethylacetamide, N,N-dimethylformamide, dimethylsulfoxide, sulfolane, hexamethylphosphoric triamide, 1,3-dimethyl-2-imidazolidinone; aliphatic and aromatic hydrocarbons such as n-pentane, n-hexane, n-octane, cyclohexane, benzene, toluene, xylene, tetralin, naphthalene, chlorobenzene, chlorotoluene, o-dichlorobenzene, and 1-chloronaphthalene; ethers such as diethyl ether, tetrahydrofuran, 1,4-dioxane, ethylene glycol dimethyl ether, triethylene glycol dimethyl ether, polyethylene glycol, polypropylene glycol, diglyme, anisole, phenetole, and diphenyl ether; ketones such as acetone, methyl ethyl ketone, diisopropyl ketone, and benzophenone; tertiary amines such as tributylamine, N,N-dimethylaniline, pyridine, and quinoline; nitro compounds such as nitromethane, nitroethane, nitrobenzene, and o-nitrotoluene; nitriles such as acetonitrile, propionitrile, 1,2-dicyanoethane, and benzonitrile; alcohols such as methanol, ethanol, n-propanol, 2-propanol, n-butanol, and tert-butyl alcohol; chloroform; carbon tetrachloride; dichloromethane; and water.

These solvents may be used alone or in combination. Solvents commercially available may be directly used or may be refined, e.g., distilled, before use.

In using the solvent during the reaction, the content of the solvent is not particularly limited. The content can be adjusted based on the reaction conditions, such as the type and the content of starting materials, the reaction temperature, and the reaction pressure. In general, 1 to 50 grams of solvent is used per gram of the halogenated compound.

The content of the compound represented by general formula (1), the content of the compound represented by general formula (2), and the content of the halogenated compound used in the reaction can be suitably adjusted

based on the reaction conditions, such as the type of compound represented by general formula (1), the type of compound represented by general formula (2), the type of halogenated compound, the type and the content of the solvent when the solvent is used, the reaction temperature, and the reaction pressure. In general, the molar ratio of the halogen atoms in the halogenated compound to the compound represented by general formula (2) to the compound represented by general formula (1) is in the range of 1.0:0.5:0.001 to 1.0:4.0:0.999.

The reaction is preferably conducted under an inert gas atmosphere such as nitrogen, argon, or carbon dioxide but can be conducted in air.

Conditions such as the reaction temperature, the reaction time, and the reaction pressure are not particularly limited as long as the target substance can be produced.

The reaction temperature is typically in the range of 0 to 250°C, preferably 0 to 230°C, and more preferably 30 to 200°C.

The reaction time is typically 48 hours or less, preferably in the range of 0.01 to 30 hours, and more preferably in the range of 0.02 to 15 hours.

The reaction pressure is typically in the range of 0.01 to 8 atm, preferably 0.1 to 5 atm, and more preferably 1.0 to 3 atm.

It was found that during the reaction between the compound represented by general formula (2) and the halogenated compound in the presence of a compound represented by general formula (1), the reaction rate can be increased and the reaction can progress more efficiently when a compound represented by general formula (10) exists in the system:

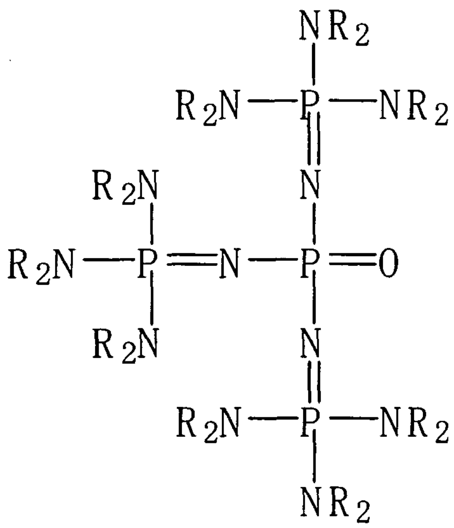

(10)

(wherein $R_2$ is the same or different; $R_2$ each independently represent a $C_1$-$C_{10}$ hydrocarbon group or two $R_2$ on the same nitrogen atom may be bonded with each other to form a ring with the nitrogen atom) .

In general formula (10), $R_2$ represent the same groups as the examples of $R_2$ in general formula (1). Preferable examples of $R_2$ are also the same as those of $R_2$ in general formula (1).

Compounds represented by general formula (10) can be prepared by a method disclosed in G.N. Koidan et al., Journal of General Chemistry of the USSR, vol. 55, p. 1453 (1985) or a method comparable to this method.

The content of the compound represented by general formula (10) can be adjusted according to the reaction conditions such as the type and the content of compound represented by general formula (1), the type and the content of compound represented by general formula (2), the type and the content of the halogenated compound, the type and the content of the solvent when the solvent is used, the reaction temperature, and the reaction pressure. Typically, the content of the compound represented by general formula (10) is less than 1 mole, preferably 0.001 to 0.1 mole, and more preferably 0.01 to 0.05 mole per mole of the halogen atom (X) in the halogenated compound to be substituted.

The target compound of the present invention prepared by the reaction described above can be recovered from the reaction mixture by a known process, such as extraction, distillation, recrystallization, or column chromatography, upon completion of the reaction.

The compound represented by general formula (1) can be recovered from the reaction mixture by, for example, a known separation process after the target compound is recovered and can be reused.

EXAMPLES

The present invention will now be described by way of nonlimiting examples. The yields described in the examples and comparative examples below are yields with respect to the halogenated compound used and are determined by gas chromatography (hereinafter, simply "GC") unless otherwise noted. GC-9A manufactured by Shimadzu Corporation was used as the GC apparatus, and G-250 manufactured by Chemicals Evaluation and Research Institute was used as the column.

[EXAMPLE 1]

A 50-ml flask equipped with a thermometer and a cooling tube was charged with 0.96 g (9.00 mmol) of 3-chlorotetrahydrofuran, 0.66 g (13.5 mmol) of sodium cyanide, 0.70 g (0.90 mmol) of a phosphazenium compound, i.e., tetrakis[tris(dimethylamino)phosphoranylideneamino] phosphonium chloride ($[(Me_2N)_3P=N]_4P^+$, $Cl^-$), thoroughly dried with flowing dry nitrogen at 100°C, and 9.44 g of anhydrous N,N-dimethylformamide (hereinafter, simply referred to as the "DMF") under a nitrogen atmosphere. The temperature of the resulting suspension was increased to 130°C in about 20 minutes while stirring. A trace amount of the reaction mixture was sampled after one hour, four hours, and six hours to conduct quantitative analysis by GC. The yield of 3-cyanotetrahydrofuran corresponding to the three reaction times was 19%, 73%, and 85%, respectively. The same reaction was separately conducted under the same conditions. The resulting reaction mixture was cooled to room temperature, and a deposited solid matter was separated by filtering. The solid matter was washed twice with 4 ml of DMF, and the wash and the filtrate were mixed. The resulting solution was extracted three times with 50 ml of diethyl ether. The ether phase was distilled under a reduced pressure to obtain 0.66 g of nearly pure 3-cyanotetrahydrofuran in the form of oily matter.

[COMPARATIVE EXAMPLE 2]

The reaction and the quantitative analysis were conducted as in EXAMPLE 1 but without the phosphazenium compound, i.e., tetrakis [tris(dimethylamino)phosphoranylideneamino] phosphonium chloride. The yield of 3-cyanotetrahydrofuran after one hour, four hours, and six hours was 8%, 28%, and 40%, respectively.

[COMPARATIVE EXAMPLE 2]

The reaction and the quantitative analysis were conducted as in EXAMPLE 1 except that an equimolar of tetraphenylphosphonium chloride was used instead of the phosphazenium compound, i.e., tetrakis [tris(dimethylamino) phosphoranylideneamino] phosphonium chloride. The yield of 3-cyanotetrahydrofuran after one hour, four hours, and six hours was 7%, 25%, and 37%, respectively.

[COMPARATIVE EXAMPLE 3]

The reaction and the quantitative analysis were conducted as in EXAMPLE 1 except that an equimolar of 18-crown-6-ether was used instead of the phosphazenium compound, i.e., tetrakis [tris(dimethylamino)phosphoranylideneamino] phosphonium chloride. The yield of 3-cyanotetrahydrofuran after one hour, four hours, and six hours was 10%, 34%, and 45%, respectively.

[EXAMPLE 2]

A reaction was conducted as in EXAMPLE 1 except that 3-(chloromethyl)tetrahydrofuran was used instead of the 3-chlorotetrahydrofuran, sodium azide was used instead of the sodium cyanide, the reaction temperature was changed to 100°C, and the reaction time was changed to 4 hours without tracing the progress. The yield of 3-(azidomethyl)tetrahydrofuran was 85%.

[EXAMPLES 3 to 10]

A reaction was conducted as in EXAMPLE 1 except that various halogenated compounds and various metal compounds shown in Table 1 were used instead of 3-chlorotetrahydrofuran and sodium cyanide of EXAMPLE 1, that the reaction temperature and the reaction time were changed as in Table 1, and that the progress of the reaction was

not traced. The results are shown in Table 1.

[EXAMPLE 11]

A 50-ml pressure glass vessel equipped with a thermometer was charged with 1.25 g (12.0 mmol) of chlorocyclopentane, 1.05 g (18.0 mmol) of potassium fluoride, 0.93 g (1.20 mmol) of a phosphazenium compound, i.e., tetrakis [tris(dimethylamino)phosphoranylideneamino] phosphonium chloride ($[(Me_2N)_3P{=}N]_4P^+$, $Cl^-$), thoroughly dried with flowing dry nitrogen at 100°C, and 9.44 g of anhydrous DMF under a nitrogen atmosphere. The temperature of the resulting suspension was increased to 110°C in about 10 minutes while stirring. The reaction was continued for six hours at 110°C. Subsequently, a trace amount of the reaction mixture was sampled to conduct quantitative analysis by GC. The yield of fluorocyclopentane was 68%.

[EXAMPLES 12 to 14]

A reaction was conducted as in EXAMPLE 11 except that various halogenated compounds and various metal compounds shown in Table 2 were used instead of chlorocyclopentane and potassium fluoride and that the reaction temperature and the reaction time were changed as in Table 2. The results are shown in Table 2.

[EXAMPLE 15]

A 50-ml flask equipped with a thermometer and a cooling tube was charged with 1.53 g (10 mmol) of trans-1,2-dichlorocyclohexane, 0.74 g (15 mmol) of sodium cyanide, 0.78 g (1.00 mmol) of a phosphazenium compound, i.e., tetrakis [tris(dimethylamino)phosphoranylideneamino] phosphonium chloride ($[(Me_2N)_3P{=}N]_4P^+$, $Cl^-$), thoroughly dried with flowing dry nitrogen at 100°C, and 12.5 g of anhydrous 1,3-dimethyl-2-imidazolidinone (hereinafter referred to as "DMI") under a nitrogen atmosphere. The temperature of the resulting suspension was increased to 150°C in about 25 minutes while stirring, and the reaction was continued for five hours at 150°C. Subsequently, a trace amount of the reaction mixture was sampled to conduct quantitative analysis by GC. The yield of 1,2-dicyanocyclohexane (a mixture of cis and trans configurations) was 51%.

[EXAMPLE 16]

A 50-ml flask equipped with a thermometer and a cooling tube was charged with 0.96 g (9.00 mmol) of 3-chlorotetrahydrofuran, 0.66 g (13.5 mmol) of sodium cyanide, 0.70 g (0.90 mmol) of a phosphazenium compound, i.e., tetrakis [tris(dimethylamino)phosphoranylideneamino] phosphonium chloride ($[(Me_2N)_3P{=}N]_4P^+$, $Cl^-$), thoroughly dried with flowing dry nitrogen at 100°C, 0.08 g (0.14 mmol) of tris[tris(dimethylamino)phosphoranylideneamino]phosphine oxide ($[(Me_2N)_3P{=}N]_3P{=}O$), thoroughly dried with flowing dry nitrogen at 80°C, and 9.44 g of anhydrous DMF under a nitrogen atmosphere. The temperature of the resulting suspension was increased to 130°C in about 20 minutes while stirring. A trace amount of the reaction mixture was sampled after one hour, four hours, and six hours to conduct quantitative analysis by GC. The yield of 3-cyanotetrahydrofuran corresponding to the three reaction times was 25%, 87%, and 96%, respectively. The same reaction was separately conducted under the same conditions. The resulting reaction mixture was cooled to room temperature, and a deposited solid matter was separated by filtering. The solid matter was washed twice with 4 ml of DMF, and the wash and the filtrate were mixed. The resulting solution was extracted three times with 50 ml of diethyl ether. The ether phase was distilled under a reduced pressure to obtain 0.75 g of nearly pure 3-cyanotetrahydrofuran in the form of oily matter.

[EXAMPLE 17]

A reaction is conducted as in EXAMPLE 16 except that 3-(chloromethyl)tetrahydrofuran was used instead of 3-chlorotetrahydrofuran, sodium azide was used instead of sodium cyanide, the reaction temperature was changed to 100°C, and the reaction time was changed to 4 hours without tracing the progress. The yield of 3-(azidomethyl)tetrahydrofuran was 98%.

[EXAMPLES 18 to 33]

A reaction was conducted as in EXAMPLE 16 except that various halogenated compounds and various metal compounds shown in Table 3 were used instead of 3-chlorotetrahydrofuran and sodium cyanide, that the reaction temperature and the reaction time were changed as in Table 3, and that the progress of the reaction was not traced. The results are shown in Table 3.

[EXAMPLE 34]

A 50-ml pressure glass vessel equipped with a thermometer was charged with 1.25 g (12.0 mmol) of chlorocyclopentane, 1.05 g (18.0 mmol) of potassium fluoride, 0.93 g (1.20 mmol) of a phosphazenium compound, i.e., tetrakis [tris(dimethylamino)phosphoranylideneamino] phosphonium chloride ($[(Me_2N)_3P=N]_4P^+$, $Cl^-$), thoroughly dried with flowing dry nitrogen at 100°C, 0.10 g (o.17 mmol) of tris[tris(dimethylamino)phosphoranylideneamino]phosphine oxide ($[(Me_2N)_3P=N]_3P=O$), thoroughly dried with flowing dry nitrogen at 80°C, and 9.44 g of anhydrous DMF under a nitrogen atmosphere. The temperature of the resulting suspension was increased to 110°C in about 10 minutes while stirring. The reaction was continued for six hours at 110°C. Subsequently, a trace amount of the reaction mixture was sampled to conduct quantitative analysis by GC. The yield of fluorocyclopentane was 80%.

[EXAMPLE 35]

A reaction was conducted as in EXAMPLE 34 except that 2-chlorohexane was used instead of chlorocyclopentane of EXAMPLE 34 and that sodium cyanide was used instead of potassium fluoride. The yield of 2-cyanohexane was 90%.

[EXAMPLE 36]

A reaction was conducted as in EXAMPLE 34 except that 3-chloro-1-butyne was used instead of 3-chlorotetrahydrofuran in EXAMPLE 34, sodium acetate was used instead of sodium cyanide, the reaction temperature was changed to 80°C, and the reaction time was changed to 5 hours without tracing the progress. The yield of 1-methyl-2-propynyl acetate was 80%.

[EXAMPLE 37]

A 50-ml flask equipped with a thermometer, a cooling tube, and a dropping funnel was charged with 0.34 g (13.9 mmol) of sodium hydride and 15.0 g (126 mmol) of chlorocyclohexane under a nitrogen atmosphere. Subsequently, a solution prepared by dissolving 2.40 g (15.0 mmol) of ethyl malonate in 5 ml of a chlorocyclohexane solution was added dropwise over 30 minutes while cooling the flask with an ice bath. Upon completion of the dropping, a solution prepared by dissolving 0.77 g (0.99 mmol) of a phosphazenium compound, i.e., tetrakis [tris(dimethylamino) phosphoranylideneamino] phosphonium chloride ($[(Me_2N)_3P=N]_4P^+$, $Cl^-$), thoroughly dried with flowing dry nitrogen at 100°C and 0.09 g (0.16 mmol) of tris[tris(dimethylamino)phosphoranylideneamino]phosphine oxide ($[(Me_2N)_3P=N]_3P=O$), thoroughly dried with flowing dry nitrogen at 80°C, in 5 ml of a chlorocyclohexyl solution was added. The temperature of the resulting suspension was increased to 130°C in about 20 minutes while stirring. The reaction was continued for 5 hours at 130°C. Subsequently, a trace amount of the reaction mixture was sampled to conduct quantitative analysis by GC. The yield of ethyl 2-cyclohexylmalonate with respect to the sodium hydride was 80%.

[Example 38]

A 50-ml flask equipped with a thermometer and a cooling tube was charged with 1.53 g (10 mmol) of trans-1,2-dichlorocyclohexane, 0.74 g (15 mmol) of sodium cyanide, 0.78 g (1.00 mmol) of a phosphazenium compound, i. e., tetrakis [tris(dimethylamino)phosphoranylideneamino] phosphonium chloride ($[(Me_2N)_3P=N]_4P^+$, $Cl^-$), thoroughly dried with flowing dry nitrogen at 100°C, 0.06 g (0.10 mmol) of tris[tris(dimethylamino)phosphoranylideneamino]phosphine oxide ($[(Me_2N)_3P=N]_3P=O$) thoroughly dried with flowing dry nitrogen at 80°C, and 12.5 g of anhydrous DMI under a nitrogen atmosphere. The temperature of the resulting suspension was increased to 150°C in about 25 minutes while stirring. The reaction was continued for 5 hours at 150°C. Subsequently, a trace amount of the reaction mixture was sampled to conduct quantitative analysis by GC. The yield of 1,2-dicyanocyclohexane (mixture of cis and trans configurations) was 62%.

[EXAMPLE 39]

A 100-ml flask equipped with a thermometer and a cooling tube was charged with 1.92 g (18.0 mmol) of 3-chlorotetrahydrofuran, 1.32 g (13.5 mmol) of sodium cyanide, 1.00 g (55.6 mmol) of water, 1.40 g (1.80 mmol) of a phosphazenium compound, i.e., tetrakis [tris(dimethylamino)phosphoranylideneamino] phosphonium chloride ($[(Me_2N)_3P=N]_4P^+$, $Cl^-$), thoroughly dried with flowing dry nitrogen at 100°C, 0.16 g (0.28 mmol) of tris[tris(dimethylamino) phosphoranylideneamino]phosphine oxide ($[(Me_2N)_3P=N]_3P=O$) thoroughly dried with flowing dry nitrogen at 80°C,

and 18.9 g of DMF under a nitrogen atmosphere. The temperature of the resulting suspension was increased to 120°C in about 16 minutes while stirring. The reaction was continued for 7 hours at 120°C. Subsequently, a trace amount of the reaction mixture was sampled to conduct quantitative analysis by GC. The yield of 3-cyanotetrahydrofuran was 95%.

[EXAMPLE 40]

A 50-ml pressure glass vessel equipped with a thermometer was charged with 1.25 g (12.0 mmol) of chlorocyclopentane, 0.88 g (18.0 mmol) of sodium cyanide, 0.93 g (1.20 mmol) of a phosphazenium compound, i.e., tetrakis [tris(dimethylamino)phosphoranylideneamino] phosphonium chloride ($[(Me_2N)_3P{=}N]_4P^+$, $Cl^-$), thoroughly dried with flowing dry nitrogen at 100°C, 0.10 g (0.17 mmol) of tris[tris(dimethylamino)phosphoranylideneamino]phosphine oxide ($[(Me_2N)_3P{=}N]_3P{=}O$) thoroughly dried with flowing dry nitrogen at 80°C, and 7.03 g of anhydrous n-octane under a nitrogen atmosphere. The temperature of the resulting suspension was increased to 110°C in about 10 minutes while stirring. The reaction was continued for 6 hours at 110°C. Subsequently, a trace amount of the reaction mixture was sampled to conduct quantitative analysis by GC. The yield of cyanopentane was 82%.

[EXAMPLE 41]

A 50-ml flask equipped with a thermometer and a cooling tube was charged with 1.00 g (9.00 mmol) of 3-(chloromethyl)tetrahydrofuran, 0.66 g (13.5 mmol) of sodium cyanide, 0.70 g (0.90 mmol) of a phosphazenium compound, i.e., tetrakis [tris(dimethylamino)phosphoranylideneamino] phosphonium chloride ($[(Me_2N)_3P{=}N]_4P^+$, $Cl^-$), thoroughly dried with flowing dry nitrogen at 100°C, 0.08 g (0.14 mmol) of tris[tris(dimethylamino)phosphoranylideneamino]phosphine oxide ($[(Me_2N)_3P{=}N]_3P{=}O$) thoroughly dried with flowing dry nitrogen at 80°C, and 8.65 g of anhydrous toluene. The temperature of the resulting suspension was increased to 100°C in about 10 minutes while stirring. The reaction was continued for 5 hours at 100°C. Subsequently, a trace amount of the reaction mixture was sampled to conduct quantitative analysis by GC. The yield of 3-(cyanomethyl)tetrahydrofuran was 90%.

[EXAMPLE 42]

A 50-ml pressure glass vessel equipped with a thermometer was charged with 1.62 g (12.0 mmol) of 4-chloroheptane, 0.88 g (18.0 mmol) of sodium cyanide, 0.98 g (1.20 mmol) of a phosphazenium compound, i.e., tetrakis [tris(dimethylamino)phosphoranylideneamino] phosphonium bromide ($[(Me_2N)_3P{=}N]_4P^+$, $Br^-$) , thoroughly dried with flowing dry nitrogen at 100°C, 0.11 g (0.19 mmol) of tris[tris(dimethylamino)phosphoranylideneamino]phosphine oxide ($[(Me_2N)_3P{=}N]_3P{=}O$), and 9.44 g of anhydrous DMF under a nitrogen atmosphere. The temperature of the resulting suspension was increased to 110°C in about 10 minutes while stirring. The reaction was continued for 6 hours at 110°C. Subsequently, a trace amount of the reaction mixture was sampled to conduct quantitative analysis by GC. The yield of 4-cyanoheptane was 89%.

[EXAMPLE 43]

A 100-ml flask equipped with a thermometer and a cooling tube was charged with 2.13 g (20.0 mmol) of 2-chlorotetrahydrofuran, 6.00 g (40.0 mmol) of sodium iodide, 2.22 g (2.00 mmol) of a phosphazenium compound, i.e., tetrakis [tris(diethylamino)phosphoranylideneamino] phosphonium chloride ($[(Et_2N)_3P{=}N]_4P^+$,$Cl^-$), thoroughly dried with flowing dry nitrogen at 100°C, 0.25 g (0.43 mmol) of tris[tris(dimethylamino)phosphoranylideneamino]phosphine oxide ($[(Me_2N)_3P{=}N]_3P{=}O$) thoroughly dried with flowing dry nitrogen at 80°C, and 28.3 g of anhydrous DMF under a nitrogen atmosphere. The temperature of the resulting suspension was increased to 100°C in about 10 minutes while stirring. The reaction was continued for 4 hours at 100°C. Subsequently, a trace amount of the reaction mixture was sampled to conduct quantitative analysis by GC. The yield of 2-iodotetrahydrofuran was 93%.

[EXAMPLE 44]

A reaction was conducted as in EXAMPLE 43 except that chlorodiphenylmethane was used instead of 2-chlorotetrahydrofuran of EXAMPLE 43, that sodium hydroxide was used instead of sodium iodide, that the reaction temperature was changed to 110°C, and that the reaction time was changed to 7 hours. The yield of benzhydrol was 87%.

[EXAMPLE 45]

A reaction was conducted as in EXAMPLE 43 except that chlorocyclohexane was used instead of 2-chlorotetrahydrofuran, that sodium phenoxide was used instead of sodium iodide, that tetrakis [tri(pyrrolidin-1-yl)phospho-

ranylideneamino]phosphonium chloride ($(Py_3P=N)_4P^+$, $Cl^-$) was used instead of tetrakis [tris(diethylamino)phosphoranylideneamino] phosphonium chloride, that the reaction temperature was changed to 120°C, and that the reaction time was changed to 6 hours. The yield of cyclohexylphenyl ether was 81%.

[EXAMPLE 46]

A 100-ml flask equipped with a thermometer and a cooling tube was charged with 1.92 g (18.0 mmol) of 3-chlorotetrahydrofuran, 1.32 g (27.0 mmol) of sodium cyanide, 1.40 g (1.80 mmol) of a phosphazenium compound, i.e., tetrakis [tris(dimethylamino)phosphoranylideneamino] phosphonium chloride ($[(Me_2N)_3P=N]_4P^+$, $Cl^-$), thoroughly dried with flowing dry nitrogen at 100°C, 0.16 g (0.28 mmol) of tris[tris(dimethylamino)phosphoranylideneamino]phosphine oxide ($[(Me_2N)_3P=N]_3P=O$), thoroughly dried with flowing dry nitrogen at 80°C, and 17.3 g of anhydrous toluene under a nitrogen atmosphere. The temperature of the resulting suspension was increased to 120°C in about 20 minutes while stirring. The reaction was continued for 9 hours at 120°C. Subsequently, a trace amount of the reaction mixture was sampled to conduct quantitative analysis by GC. The yield of 3-cyanotetrahydrofuran was 94%. Deposited solid matter in the reaction mixture was filtered, and the solid matter was washed twice with 4 ml of toluene, and the wash and the filtrate were mixed. 3-Chlorotetrahydrofuran, i.e., the starting material, 3-cyanotetrahydrofuran, i.e., the product, toluene and DMF were removed from this solution by distillation to obtain 1.48 g of a mixed solid matter of the phosphazenium compound and the phosphine oxide. The solid matter was added to 5.00 g of hexane, and solid precipitate (i.e., the phosphazenium compound) was separated by filtering. The solid obtained was added to 8.14 g of water and heated to 40°C to prepare a solution. The solution was cooled to 0°C with stirring to precipitate crystals. After the crystals were filtered and washed twice with 2 ml of 0°C water, the resulting crystals were dried with flowing dry nitrogen at 100°C to obtain 1.32 g of the phosphazenium compound. This phosphazenium compound recovered was reused to conduct another cycle of reaction under the same conditions, and a trace amount of the reaction mixture was sampled to conduct quantitative analysis by GC. The yield of 3-cyanotetrahydrofuran was 92%.

[Table 1]

| Example | Halogen compound | Metal compound | Reaction temperature, °C | Reaction time, hr | Product | Yield |
|---|---|---|---|---|---|---|
| 3 | 2-(chloromethyl)tetrahydrofuran | sodium methoxide | 130 | 3 | methyltetrahydrofurfuryl ether | 70% |
| 4 | ethyl 2-chlorobutyrate | sodium thiocyanide | 130 | 4 | ethyl 2-thiocyanate butyrate | 74% |
| 5 | 4-chlorotetrahydropyran | sodium hydroxide | 90 | 7 | 4-hydroxytetrahydropyran | 66% |
| 6 | α-bromo-γ-butyrolactone | sodium phenoxide | 80 | 4 | 3-phenoxydihydrofuran-2-on | 70% |
| 7 | ethyl 3-chloro-n-valerate | sodium cyanide | 130 | 9 | ethyl 3-cyano-n-valerate | 62% |
| 8 | ethyl 3-chloro-4-phenyl-n-butyrate | sodium cyanide | 130 | 10 | ethyl 3-cyano-4-phenyl-n-butyrate | 58% |
| 9 | ethyl 4-benzyloxy-3-chloro-n-butyrate | sodium cyanide | 130 | 10 | ethyl 4-benzyloxy-3-cyano-n-butyrate | 61% |
| 10 | ethyl 3-chloro-5-methyl-n-caproate | sodium cyanide | 130 | 12 | ethyl 3-cyano-5-methyl-n-caproate | 65% |

[Table 2]

| Example | Halogen compound | Metal compound | Reaction temperature, °C | Reaction time, hr | Product | Yield |
|---|---|---|---|---|---|---|
| 12 | 2-chlorohexane | sodium cyanide | 110 | 4.5 | 2-cyanohexane | 75% |
| 13 | 3-chloro-2-butanone | sodium n-butylthiolate | 120 | 5 | 3-(n-butylthio)-2-butanone | 74% |
| 14 | α-methylbenzyl chloride | sodium propionate | 100 | 4 | 1-phenylethyl propionate | 72% |

[Table 3]

| Example | Halogen compound | Metal compound | Reaction temperature, °C | Reaction time, hr | Product | Yield |
|---|---|---|---|---|---|---|
| 18 | chlorocycloheptane | potassium fluoride | 110 | 6 | fluorocycloheptane | 85% |
| 19 | 1-chloro-3,3-dimethyl-2-butanone | sodium cyanide | 130 | 6 | 1-fluoro-3,3-dimethyl-2-butanone | 65% |
| 20 | 2-chloroacetophenone | potassium fluoride | 120 | 6 | 2-fluoroacetophenone | 60% |
| 21 | 2-chloroacetonitrile | sodium methoxide | 120 | 6 | 2-methoxyacetonitrile | 64% |
| 22 | 1-chloro-1-nitropropane | potassium nitrite | 100 | 7 | 1,1-dinitropropane | 70% |
| 23 | chloromethyl butyrate | potassium fluoride | 100 | 6 | fluoromethyl butyrate | 72% |
| 24 | methyl 2-chloro-2-phenylacetate | sodium n-butylthiolate | 90 | 6 | methyl 2-butylsulfanil-2-phenyl acetate | 83% |
| 25 | 9-chlorofluorene | lithium diethylamide | 110 | 5 | 9-(diethylamino)fluorene | 90% |
| 26 | methyl 3-chlorobutyrate | sodium thiocyanide | 100 | 5 | methyl 3-thiocyanate butyrate | 85% |
| 27 | 4-chlorotetrahydropyran | sodium hydroxide | 90 | 8 | 4-hydroxytetrahydropyran | 75% |
| 28 | 2-chloropropionitrile | sodium hydroxide | 120 | 8 | 2-hydroxypropionitrile | 70% |
| 29 | α-bromo-γ-butyrolactone | sodium phenoxide | 80 | 4 | 3-phenoxydihydrofuran-2-on | 80% |
| 30 | ethyl 3-chloro-n-valerate | sodium cyanide | 130 | 9 | ethyl 3-cyano-n-valerate | 68% |
| 31 | ethyl 3-chloro-4-phenyl-n-butyrate | sodium cyanide | 130 | 10 | ethyl 3-cyano-4-phenyl-n-butyrate | 65% |
| 32 | ethyl 4-benzyloxy-3-chloro-n-butyrate | sodium cyanide | 130 | 10 | ethyl 4-benzyloxy-3-cyano-n-butyrate | 68% |
| 33 | ethyl 3-chloro-5-methyl-n-caproate | sodium cyanide | 130 | 12 | ethyl 3-cyano-5-methyl-n-caproate | 70% |

## Claims

**1.** A method for producing an organic compound having Q, the method comprising:

reacting a compound represented by general formula (2) with an organic starting material having at least one halogen atom bonded to a carbon atom having four σ bonds so as to replace the halogen atom in the organic starting material with Q:

$$MQ_a \quad (2)$$

(wherein M represents an alkali metal atom, an alkali earth metal atom, or a rare earth metal atom; Q represents a moiety of an inorganic acid or an active hydrogen compound derived by eliminating a proton, wherein Q is a halogen atom different from the halogen atom in the organic starting material having the halogen atom bonded to the carbon atom having the four σ bonds; and a represents an integer of 1 to 3) in the presence of a compound represented by general formula (1)

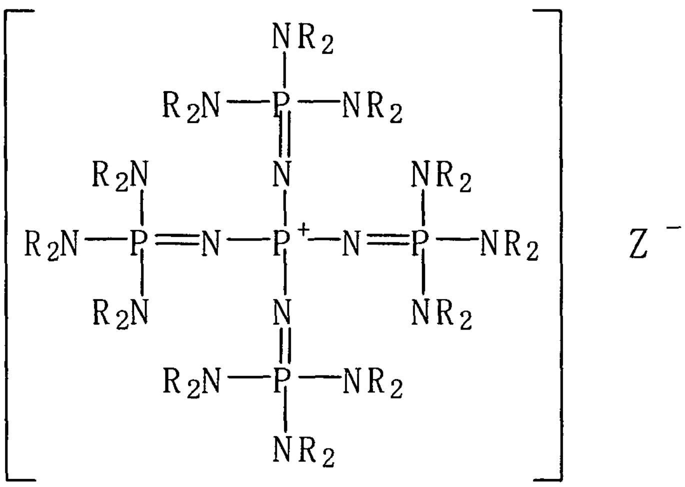

(1)

(wherein $Z^-$ represents an anion derived by eliminating a proton from an inorganic acid or an active hydrogen compound; $R_2$ is the same or different; $R_2$ each independently represent a $C_1$-$C_{10}$ hydrocarbon group or two $R_2$ on the same nitrogen atom may be bonded with each other to form a ring with the nitrogen atom).

**2.** The method according to claim 1, wherein the organic starting material having the halogen atom bonded with the carbon atom having the four σ bonds is represented by general formula (3), and the organic compound having Q is represented by general formula (4):

$$B\text{-}X \quad (3)$$

(wherein X represents a halogen atom, and B represents an organic group),

$$B\text{-}Q \quad (4)$$

(wherein B and Q are the same as above).

**3.** The method according to claim 2, wherein B in the compound represented by general formula (3) is selected from a $C_1$-$C_{12}$ straight or branched alkyl group, a group represented by general formula (5), a group represented by general formula (6), a group represented by general formula (7), a group represented by general formula (8), a

group represented by general formula (9), or a $C_1$-$C_{12}$ straight or branched alkyl group substituted with a group selected from Substituent Group α:

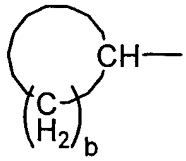

(5)

(wherein b is an integer of 2 to 9),

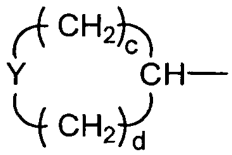

(6)

(wherein Y represents an oxygen atom, a sulfur atom, or NR'; c and d represent integers satisfying the relationship c + d = 3 to 6; and R' in NR' represents a hydrogen atom or a methyl group):

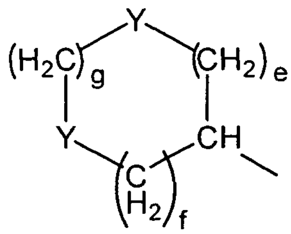

(7)

(wherein Y represents an oxygen atom, a sulfur atom, or NR'; e, f, and g are integers satisfying the relationship e + f + g = 2 to 5; and R' in NR' represents a hydrogen atom or a methyl group),

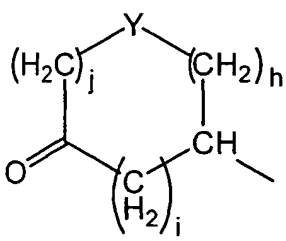

(8)

(wherein Y represents an oxygen atom, a sulfur atom, or NR'; h, i and j represent integers satisfying the relationship h + i + j = 2 to 5; and R' in NR' represents a hydrogen atom or a methyl group),

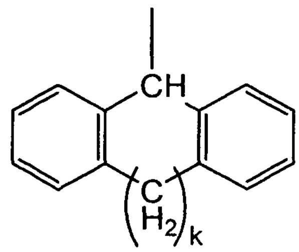

(9)

(wherein k represents an integer between 0 and 2),
[Substituent Group α]
groups represented by general formula (5) to (9); $C_2$-$C_{10}$ alkenyl groups; $C_2$-$C_{10}$ alkynyl groups; $C_6$-$C_{12}$ aryl groups; acyl groups having a total of 2 to 10 carbon atoms including carbon atoms of carbonyl groups; acyloxy groups having a total of 2 to 10 carbon atoms including carbon atoms of carbonyl groups; alkoxycarbonyl groups having a total of 2 to 10 carbon atoms including carbon atoms of carbonyl groups; arylcarbonyl groups having a total of 7 to 10 carbon atoms including carbon atoms of carbonyl groups; alkoxycarbonylalkyl groups having a total of 3 to 10 carbon atoms including carbon atoms of carbonyl groups; nitro groups; and cyano group.

**4.** The method according to claim 3, wherein all $R_2$ in the compound represented by general formula (1) in claim 1 are $C_1$-$C_2$ alkyl groups or every pair of $R_2$ bonded on the same nitrogen atom forms a ring as a $C_4$-$C_5$ alkylene group.

**5.** The method according to claim 3, wherein the compound from which $Z^-$ in general formula (1) is derived is a hydrogen halide.

**6.** The method according to claim 3, wherein Q in the compound represented by general formula (2) in claim 1 is a moiety derived by eliminating a proton of one selected from hydrogen halides, hydrogen cyanides, hydrogen azides, thiocyanic acids, malonic esters, acetoacetic esters, cyanoacetic esters, water, carboxylic acids having a total of 1 to 20 carbon atoms including carbon atoms of carbonyl groups, $C_1$-$C_{20}$ alcohols, $C_6$-$C_{20}$ aromatic compounds having 1 to 3 hydroxyl groups, aliphatic secondary amines having a total of 2 to 20 carbon atoms, aromatic secondary amines having a total of 6 to 20 carbon atoms, $C_1$-$C_{10}$ monovalent thiols, and $C_1$-$C_{10}$ aromatic mercapto compounds.

**7.** The method according to claim 3, wherein, in the compound represented by general formula (1) in claim 1, all $R_2$ in general formula (1) are methyl or ethyl groups, or every pair of $R_2$ bonded to the same nitrogen atom is a tetramethylene group; $Z^-$ is an chlorine anion or a bromine anion; in the compound represented by general formula (2) of claim 1, Q is a moiety of an active hydrogen compound derived by eliminating a proton, the active hydrogen compound being selected from Compound Group β; in the compound represented by general formula (3) in claim 2, B represents a $C_1$ to $C_{10}$ straight alkyl group; b in the group represented by general formula (5) in claim 3 is 4 to 7; Y in the group represented by general formula (6) in claim 3 is an oxygen atom, and c + d is 3 or 4; in the group represented by general formula (8) in claim 3, Y is an oxygen atom, h = 2, i = 0, and j = 0; k in the group represented by general formula (9) in claim 3, k is zero; or $C_1$-$C_{10}$ straight alkyl groups substituted with groups selected from Substituent Group α in claim 3 consists of Substituent Group γ:
[Compounds Group β]
hydrogen fluoride, hydrogen iodide, hydrogen cyanide, hydrogen azide, thiocyanic acid, diethyl malonate, water, acetic acid, methanol, phenol, diethylamine, nitrous acid, and n-butylthiol;
[Substituent Group γ]
tetrahydrofuryl group, 2-oxotetrahydrofuryl group, ethynyl group, phenyl group, acetyl group, pivalyl group, benzoyl group, butyryloxy group, methoxycarbonyl group, ethoxycarbonyl group, methoxycarbonylmethyl group, nitro group, and cyano group.

**8.** The method according to one of claims 1 to 7, wherein the reaction is conducted in the presence of a compound represented by general formula (10):

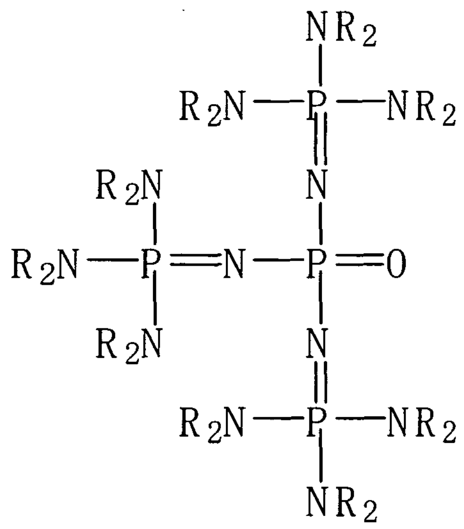

(10)

(wherein $R_2$ is the same or different; $R_2$ each independently represent a $C_1$-$C_{10}$ hydrocarbon group or two $R_2$ on the same nitrogen atom may be bonded with each other to form a ring with the nitrogen atom).

**9.** The method according to one of claims 3 to 8, wherein, in the compound represented by general formula (10) in claim 8, all $R_2$ are $C_1$-$C_2$ alkyl groups, or every pair $R_2$ bonded to the same nitrogen atom forms a ring as a $C_4$-$C_5$ alkylene group.

European Patent Office

# EUROPEAN SEARCH REPORT

Application Number
EP 04 25 3380

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,A | EP 1 275 630 A (MITSUI CHEMICALS INC) 15 January 2003 (2003-01-15) * the whole document * | 1 | C07B39/00 C07B37/04 C07B41/00 C07B43/00 |
| A | JENNER G: "Effect of pressure on sterically congested cyanoalkylation reactions of alcohols" TETRAHEDRON, ELSEVIER SCIENCE PUBLISHERS, AMSTERDAM, NL, vol. 58, no. 21, 20 May 2002 (2002-05-20), pages 4311-4317, XP004357405 ISSN: 0040-4020 * the whole document * | 1 | C07D307/24 C07D307/14 C07C17/20 C07C253/14 C07C255/19 C07C331/18 C07D307/32 C07C319/14 C07C45/63 C07C205/02 C07C209/08 C07F9/06 |

TECHNICAL FIELDS SEARCHED (Int.Cl.7)

C07B
C07D
C07C
C07F

The present search report has been drawn up for all claims

| Place of search | Date of completion of the search | Examiner |
|---|---|---|
| The Hague | 24 September 2004 | Diederen, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 04 25 3380

This annex lists the patent family members relating to the patent documents cited in the above-mentioned European search report.
The members are as contained in the European Patent Office EDP file on
The European Patent Office is in no way liable for these particulars which are merely given for the purpose of information.

24-09-2004

| Patent document cited in search report | Publication date | Patent family member(s) | Publication date |
|---|---|---|---|
| EP 1275630 A | 15-01-2003 | EP 1275630 A1 | 15-01-2003 |
| | | US 2002161227 A1 | 31-10-2002 |
| | | CN 1380880 T | 20-11-2002 |
| | | WO 0181274 A1 | 01-11-2001 |
| | | JP 2002003427 A | 09-01-2002 |

EPO FORM P0459

For more details about this annex : see Official Journal of the European Patent Office, No. 12/82